# EUROPEAN PATENT APPLICATION

(11) **EP 1 460 700 A2**
(43) Date of publication of application: **22.09.2004**
(21) Application number: 04251586.6
(22) Date of filing: 19.03.2004
(51) Int. Cl.: H01M 4/02, H01M 4/36, H01M 10/40, H01M 4/48, H01M 4/58

(54) **Electrode having metal vanadium oxide nanoparticles for alkali metal-containing electrochemical cells**

(30) Priority: 19.03.2003 US 391885
(71) Applicant: Wilson Greatbatch Technologies, Inc., Clarence, New York 14031 (US)
(72) Inventor: Takeuchi, Esther S., East Amherst New York 14051 (US); Leising, Randolph, Williamsville New York 14221 (US); Rubino, Robert, Williamsville New York 14221 (US); Hong, Gan, East Amherst New York 14051 (US)
(74) Representative: Colmer, Stephen Gary

(57) **Abstract**

A new cathode design having a second cathode active material of a relatively high energy density but of a relatively low rate capability sandwiched between two current collectors with a first cathode active material having a relatively low energy density but of a relatively high rate capability in contact with the opposite sides of the two current collectors, is described. At least the first cathode active material is of particles having an average diameter less than about 1µ. The present cathode design is useful for powering an implantable medical device requiring a high rate discharge application.

## Description

### BACKGROUND OF THE INVENTION

### 1. Field Of Invention

This invention relates to the conversion of chemical energy to electrical energy. In particular, the present invention relates to an electrode comprising a first active material of a relatively low energy density but of a relatively high rate capability and a second active material having a relatively high energy density but of a relatively low rate capability. The first and second active materials are short circuited to each other by contacting the opposite sides of a current collector. A preferred form of the electrode comprises nanoparticles of at least the high rate cathode active material. The increased surface area of the high rate material afforded by the nanoparticles increases the discharge rate of the cell. This is particularly important when the cell powers an implantable medical device, such as a cardiac defibrillator. In a secondary cell, the nanoparticles provide for greater cycling efficiency.

### 2. Prior Art

As is well known by those skilled in the art, an implantable cardiac defibrillator is a device that requires a power source for a generally medium rate, constant resistance load component provided by circuits performing such functions as, for example, the heart sensing and pacing functions. From time-to-time, the cardiac defibrillator may require a generally high rate, pulse discharge load component that occurs, for example, during charging of a capacitor in the defibrillator for the purpose of delivering an electrical shock to the heart to treat tachyarrhythmias, the irregular, rapid heartbeats that can be fatal if left uncorrected.

It is generally recognized that for lithium cells, silver vanadium oxide (SVO) and, in particular, ε-phase silver vanadium oxide (AgV₂O_{5.5}), is preferred as the cathode active material. This active material has a theoretical volumetric capacity of 1.37 Ah/ml. By comparison, the theoretical volumetric capacity of CFₓ (x = 1.1) is 2.42 Ah/ml, which is 1.77 times that of ε-phase silver vanadium oxide. For powering a cardiac defibrillator, SVO is preferred because it delivers high current pulses or high energy within a short period of time. Although CFₓ has higher volumetric capacity, it cannot be used in medical devices requiring a high rate discharge application due to its low to medium rate of discharge capability.

A novel electrode construction using both a high rate active material, such as SVO, and a high energy density material, such as CFₓ, is described in U.S. application Serial No. 09/560,060. This application is assigned to the assignee of the present invention and incorporated herein by reference. However, it is believed that the discharge performance of this cell is further improved by providing at least the high rate SVO material in the form of nanoparticles having an average particle size of less than 1 micron (1µ).

### SUMMARY OF THE INVENTION

Accordingly, an object of the present invention is to improve the performance of alkali metal-containing electrochemical cells, whether of a primary or a secondary chemistry, by providing a new electrode design. The electrode for the primary cell has the relatively high rate capability metal vanadium oxide nanoparticles, for example, SVO, contacted to one side of a current collector while the relatively high energy density of, for example, CFₓ, is contacted to the other side of the current collector. This design has the separate SVO and CFₓ materials short-circuited to each other through the current collector. An exemplary cathode for a primary cell may have the configuration of: SVO/current collector/CFₓ/current collector/SVO.

Providing the active materials in a short circuit relationship means that their respective attributes of high rate and high energy density benefit overall cell discharge performance. Further, at least the high rate SVO material has an average particle size of less than 1 micron. The increased surface area provided by the metal vanadium oxide nanoparticles improves the cell's discharge performance, especially during high rate pulsing, such as when the cell charges a capacitor in a cardiac defibrillator.

These and other objects of the present invention will become increasingly more apparent to those skilled in the art by reference to the following description.

### DETAILED DESCRIPTION OF THE PREFERRED EMBODIMENTS

As used herein, the term "pulse" means a short burst of electrical current of significantly greater amplitude than that of a pre-pulse current immediately prior to the pulse. A pulse train consists of at least two pulses of electrical current delivered in relatively short succession with or without open circuit rest between the pulses. An exemplary pulse train may consist of four 10-second pulses (23.2 mA/cm²) with a 15 second rest between each pulse. A typically used range of current densities for cells powering implantable medical devices is from about 15 mA/cm² to about 50 mA/cm², and more preferably from about 20 mA/cm² to about 45 mA/cm². Typically, a 10 second pulse is suitable for medical implantable applications. However, it could be significantly shorter or longer depending on the specific cell design and chemistry.

A primary electrochemical cell that possesses sufficient energy density and discharge capacity required to meet the rigorous requirements of implantable medical devices comprises an anode of a metal selected from Groups IA, IIA and IIIB of the Periodic Table of the Elements. Such anode active materials include lithium, sodium, potassium, etc., and their alloys and intermetallic compounds including, for example, Li-Si, Li-Al, Li-B and Li-Si-B alloys and intermetallic compounds. The preferred anode comprises lithium. An alternate anode comprises a lithium alloy such as a lithium-aluminum alloy. The greater the amounts of aluminum present by weight in the alloy, however, the lower the energy density of the cell.

The form of the anode may vary, but preferably it comprises a thin metal sheet or foil of the anode metal, pressed or rolled on a metallic anode current collector of titanium, titanium alloy, nickel, copper, tungsten or tantalum. The anode has an extended tab or lead of the same material as the current collector contacted by a weld to a cell case of conductive metal in a case-negative electrical configuration. Alternatively, the anode may be formed in some other geometry, such as a bobbin shape, cylinder or pellet to allow an alternate low surface cell design.

The primary electrochemical cell of the present invention further comprises a cathode of electrically conductive materials that serve as the cell's counter electrode. The cathode is preferably of solid materials and the electrochemical reaction at the cathode involves conversion of ions that migrate from the anode to the cathode into atomic or molecular forms. The solid cathode may comprise a first active material of a metal element, a metal oxide, a mixed metal oxide and a metal sulfide, and combinations thereof and a second active material, preferably of a carbonaceous chemistry or other high capacity material. The metal oxide, the mixed metal oxide and the metal sulfide of the first active material has a relatively lower energy density but a relatively higher rate capability in comparison to the second active material. A particularly preferred active material is of metal vanadium oxide nanoparticles.

A preferred preparation for metal vanadium oxide nanoparticles is by a sol-gel synthesis, as described in U.S. Patent No. 5,555,680 to Takeuchi et al. For example, if SVO is the desired metal vanadium oxide, the sol-gel preparation begins with formation of a vanadium pentoxide (V₂O₅) gel by the protonation of a vanadium species wherein the protonation may be performed, for example, by adding an acid to aqueous solutions of vanadate salts or by acidification of a vanadium salt solution via passage of the solution through a proton exchange resin. Vanadium oxide (V₂O₅) gels possess mixed valence properties as a result of reduction (typically in the range from about 1% to about 10%) of vanadium occurring during their synthesis, and also by subsequent dehydration of the synthesized gel. Formation of the vanadium pentoxide gel is also accomplished by heating a dispersed aqueous suspension of V₂O₅.

Intercalation of silver cations into the layered V₂O₅ gels is by intimate contact of a silver-containing component therewith, followed by thermal treatment. Silver cation intercalation is a proton-exchange reaction with acidic protons contained within the V₂O₅ gels. Thermal treatment of the silver vanadium oxide mixture serves, in part, to remove water from the mixture. During the dehydration process, the OH-- bonds break which, along with the intercalated cation, plays an important role in the evolution of the structural orientation of the resultant crystalline compound.

Specifically, the synthesis of SVO via sol-gel methodology uses an alkali metal hydroxide, a silver compound, and vanadium pentoxide. The alkali metal is preferably lithium while the silver component is selected from Ag, AgNO₃, AgNO₂, Ag₂O₂, AgVO₃, Ag₂CO₃, and Ag(CH₃CO₂). The materials are mixed such that the mole ratio of lithium: silver: vanadium is about 0.05: 0.95: 2.0. The mixed materials are combined with water so that the solids and/or dissolved solids range from about 5% to about 30% of the slurry, by solution weight. The resulting mixture is stirred at from about 60°C to about 90°C for about 3 hours or for a sufficient time to allow a gel to form. The gel is mixed further and then dehydrated by baking at about 375°C to 500°C for about 4 to about 48 hours to form the product silver vanadium oxide. Light grinding may be used to further comminute the SVO material to the desired nanoparticle size. U.S. Patent No. 5,555,680 to Takeuchi et al. is assigned to the assignee of the present invention and incorporated herein by reference.

Another preferred preparation for metal vanadium oxide nanoparticles is by hydrothermal synthesis. In hydrothermal synthesis, starting materials in stoichiometric molar proportions needed for the desired product active material are added to an aqueous solution and heated in a pressurized vessel past the boiling point of water. For example, if silver vanadium oxide is the desired product, suitable silver starting materials include Ag, AgNO₃, AgNO₂, Ag₂O₂, AgVO₃, Ag₂CO₃, and Ag(CH₃CO₂) while the vanadium-containing compound is selected from NH₄VO₃, AgVO₃, VO, VO_{1.27}, VO₂ , V₂O₄, V₂O₃ , V₃O₅, V₄O₉, V₆O₁₃ and V₂O₅. Typically, the temperature of hydrothermal reaction is in the range of about 120°C to about 250°C. This temperature range is much lower than the typical solid-state decomposition synthesis of about 500°C to about 1,000°C for an active material intended for use in an electrochemical reaction. Examples of hydrothermal synthesis are given in the literature: a) "Hydrothermal Synthesis of Orthorhombic LiCOₓMn₁₋ₓO₂ and Their Structural Changes During Cycling" S.-T. Myung, S. Komaba, N. Kumagai, *J. Electrochem. Soc.* **149,** A1349-A1357 (2002), and "Synthesis and reaction mechanism of 3 V LiMnO₂" Y. Nitta, M. Nagayama, H. Miyahe, A. Ohta, J. *Power Sources* **81-82,** 49-53 (1999). These publications are incorporated herein by reference.

Combustion chemical vapor deposition (CCVD) is another process for the production of metal vanadium oxide nanoparticles useful in a primary electrochemical cell. Combustion CVD is the vapor deposition of a coating onto a current collector substrate near or in a flame. This causes the reagents fed into the flame to chemically react. Flammable organic solvents such as an alkene, alkide or alcohol, containing elemental constituents of the desired coating in solution as dissolved reagents are sprayed through a nozzle and burned. Alternatively, vapor reagents are fed into the flame and burned. Likewise, non-flammable solvents are used with a gas-fueled flame. An oxidant, such as oxygen, is provided at the nozzle to react with the solvent during burning. Upon burning, reagent species in the flame chemically react and vaporize, and then deposit and form a coating on the current collector held in the combustion gases in or just beyond the flame's end. During deposition of the metal vanadium oxide nanoparticles coating, oxygen is available from at least three possible sources: the oxidant gas, the surrounding gases, and the dissolved chemical reagents. The CCVD derived coating of metal vanadium oxide nanoparticles on a current collector substrate is preferably crystalline, but may be amorphous, depending on the reagent and deposition conditions used. The resulting coatings exhibit extensive preferred orientation in X-ray diffraction patterns, evidencing that CVD occurred by heterogeneous nucleation.

Alternatively, feeding the reagent solution through a nebulizer, such as a needle bisecting a thin high velocity air stream forming a spray that is ignited and burned, performs coating deposition. Ethanol and toluene are preferred solvents.

In CCVD, the flame supplies the kinetic energy. This energy creates the appropriate thermal environment to form reactive species while coincidentally heating the substrate, thus providing the conditions for surface reactions, diffusion, nucleation, and coating growth to occur. When using combustible solutions, the solvent plays two primary roles in CCVD. First, it conveys the coating reagents into the vicinity of the current collector substrate where CVD occurs, thereby allowing the use of low cost soluble precursors. Varying the concentration of the reagents in solution and the solution flow rate produces uniform feed rates of any reagent stoichiometry. Second, combustion of the solvent produces the flame required for CCVD.

Regarding flame concepts, certain deposition conditions are preferred. First, the current collector substrate needs to be located in a zone that is sufficiently heated by the flame's radiant energy to allow surface diffusion. This temperature zone is present from about the middle of the flame to some distance beyond the flame's end. The temperature of the flame is controlled to some extent by varying the oxidant-to-fuel ratio as well as by adding non-reactive gases to the feed gas or by adding non-combustible miscible liquids to the solution.

Secondly, the metal complexes need to be vaporized and chemically changed into the desired state. For metal vanadium oxides, this occurs in the flame if sufficient oxygen is present. The high temperatures, radiant energy (infrared, ultraviolet and other radiant energy), and the plasma of the flame all aid in the reactivity of precursors. Finally, for single crystal films, the material being deposited should be in the vapor phase, and not stable particles. Particle formation can be suppressed by maintaining a low concentration of solutes, and by minimizing the distance, and therefore time, between where the reagents react and the current collector substrate location. Combining these factors means that the best CVD deposition zone is generally in the proximity of the flame's end.

Flame chemistry is a very complex phenomenon. However, flame characteristics can be controlled by: varying the gas to fuel ratio beyond stoichiometric to control the flame temperature, altering the type of fuel to effect a desired temperature, luminescence and smoking, mixing the solvents with non-flammable liquids to change the flame characteristics, decreasing the oxygen content to initialize and then increase carbon deposition, reducing droplet size to cause a liquid fuel flame to behave like a premixed gas flame because the solvents are able to vaporize prior to entering the flame, adjusting nozzle configuration and flow rates to control flame shape and velocity, and reducing the pressure because, depending on fuel and oxidizer, many flames are stable down to pressures of 10 torr.

The preferred flame temperature is from about 300°C to about 2,800°C. As flames can exist over a wide pressure range, CCVD can be accomplished at a pressure from about 10 torr to about 10,000 torr. Likewise, if plasma is formed for depositing the metal vanadium oxide nanoparticles coating, the temperature of the plasma ranges from about 800°C to about 10,000°C. The temperature of the substrate during the CCVD process also can vary depending on the type of coating desired, the current collector substrate material, and the flame characteristics. Generally, a substrate surface temperature of from about 100°C to about 2,200°C is preferred.

If droplets contact the substrate, a mixed deposition technique of both CVD and spray pyrolysis may occur. As a droplet approaches the current collector substrate, its surface may be enriched in the solutes as the solvent evaporates. The impacting drop burns off of the substrate almost instantaneously, possibly cooling and then heating this area, leaving a ring-shaped spot. The ring is thicker on the outside as more of the solutes concentrate there. This type of deposition might help increase the deposition efficiency, while maintaining heterogeneous nucleation. For a further discussion of CCVD, reference is made to U.S. Patent No. 5,652,021 to Hunt et al., which is incorporated herein by reference.

Laser pyrolysis is another method for synthesis of metal vanadium oxide nanoparticles. Laser pyrolysis relies on the production of a reactant stream containing a vanadium precursor, a radiation absorber and an oxygen source. An intense light beam, such as a laser beam, pyrolyzes the reactant stream. As the reactant stream leaves the light beam, the vanadium oxide particles are rapidly quenched. Nanoscale vanadium oxide particles produced by laser pyrolysis are subjected to heating under mild conditions in an oxygen environment or an inert environment to alter their crystal properties without destroying the nanoparticle size. Further, the stoichiometry and crystaline structure of the laser pyrolysis produced vanadium oxide nanoparticles are modified by heat processing in an oven. A thermal process then forms the metal vanadium oxide particles. A second, non-vanadium transition metal precursor, such as silver, copper and manganese, is mixed with a collection of vanadium oxide nanoparticles and heated to form the particles incorporating both metals. Under suitably mild conditions, the heat produces the desired metal vanadium oxide particles without destroying the nanoscale of the initial vanadium oxide particles. For a further discussion of the laser pyrolysis synthesis technique, reference is made to U.S. Patent No. 6,225,007 to Horne et al., which is incorporated herein by reference.

Another method for the production of metal vanadium oxide nanoparticles is by a conventional decomposition synthesis as described in U.S. Patent Nos. 4,310,609 and 4,391,729, both to Liang et al. and both assigned to the assignee of the present invention and incorporated herein by reference. These patents describe adding vanadium pentoxide to a decomposable metal salt, suitably the nitrate, of a second metal. These ingredients are thoroughly mixed and thereafter ignited. The second metal is most preferably selected from the group consisting of silver, copper, manganese and mixtures thereof. The resultant composite cathode includes V₂Oₓ wherein x ≤ 5 combined with one or more of Ag₂Oₓ wherein x = 0 to 1; CuOₓ wherein x = 0 to 1; and MnOₓ wherein x = 1 to 3.

Another synthesis technique for a metal vanadium oxide is by a combination reaction as described in U.S. Patent No. 5,221,453 to Crespi et al. This patent describes a chemical addition reaction consisting of admixing AgVO₃ and V₂O₅ in a molar ratio of 2:1 mole ratio and heating the admixture at a reaction temperature in the range of 300°C to 700°C for 5 to 24 hours. Another combination reaction consists of admixing Ag₂O and V₂O₅ in 1:2 mole ratio and heating the admixture at a reaction temperature in the range of 300°C to 700°C for 5 to 24 hours. Still another combination reaction consists of admixing Ag and V₂O₅ in a 1:1 mole ratio and heating the admixture in contact with oxygen at a reaction temperature in the range of 300°C to 700°C for 5 to 24 hours.

Still another synthesis technique for a metal vanadium oxide is described in U.S. Patent No. 5,498,494 to Takeuchi et al. (an amorphous SVO), which is assigned to the assignee of the present invention and incorporated herein by reference. This patent describes heating a mixture of phosphorous pentoxide (P₂O₅) and vanadium pentoxide (V₂O₅) at 760°C for one hour and then pouring the resulting material mixture onto a titanium foil cooled over liquid nitrogen. One of the previously described silver materials, for example, silver oxide (Ag₂O) is then added to the amorphous P₂O₅/V₂O₅ mixture with the Ag:V molar ratio being 1:2 and baked at about 400°C for about 16 hours to form silver vanadium oxide. A heated homogeneous mixture of AgV₂O₅ can also be poured into deionized water to form the amorphous SVO.

U.S. Patent No. 5,955,218 to Crespi et al. describes heat treating SVO at 390°C to 580°C after its initial synthesis, whether it be by a decomposition or a combination synthesis.

The metal vanadium oxide particles produced by the above-referenced patents Nos. 4,310,609, 4,391,729, 5,221,453, 5,498,494, and 5,955,218 are rendered to the desired nanoparticle size by passing them through an appropriately sized sieve. The metal vanadium oxide material larger than 1µ is than processed by grinding/milling it to the appropriate size. Jet milling is also an appropriate technique for rparticle size reduction. Additionally, the metal vanadium oxide particles larger than 1µ, but which were ground to 1µ or less, are reheated to a temperature in a range of about 480°C to about 550°C, preferably about 500°C for about 30 minutes to about 6 hours. This additional heating provides them with the beneficial properties of a material originally synthesized at a relatively high temperature of about 480°C to about 550°C, i.e., U.S. Patent No. 5,545,497 to Takeuchi et al., but with an average particle size less than 1µ. This patent is assigned to the assignee of the present invention and incorporated herein by reference.

One preferred metal vanadium oxide has the general formula SMₓV₂O_{y} where SM is a metal selected from Groups IB to VIIB and VIII of the Periodic Table of Elements, wherein x is about 0.30 to 2.0 and y is about 4.5 to 6.0 in the general formula. By way of illustration, and in no way intended to be limiting, one exemplary metal vanadium oxide comprises silver vanadium oxide having the general formula AgₓV₂O_{y} in any one of its many phases, i.e., β-phase silver vanadium oxide having in the general formula x = 0.35 and y = 5.8, γ-phase silver vanadium oxide having in the general formula x = 0.74 and y = 5.37 and ε-phase silver vanadium oxide having in the general formula x = 1.0 and y = 5.5, and combination and mixtures of phases thereof. For a more detailed description of such cathode active materials reference is made to the previously discussed U.S. Patent No. 4,310,609 to Liang et al.

Another preferred metal vanadium oxide cathode material includes V₂O_{z} wherein z ≤ 5 combined with Ag₂O with silver in either the silver(II), silver(I) or silver(0) oxidation state and CuO with copper in either the copper(II), copper(I) or copper(0) oxidation state to provide the mixed metal oxide having the general formula CuₓAg_{y}V₂O_{z}, (CSVO) with 0.01 ≤ z ≤ 6.5. Typical forms of CSVO are Cu_{0.16}Ag_{0.67}V₂O_{z} with z being about 5.5 and Cu_{0.5}Ag_{0.5}V₂O_{z} with z being about 5.75. The oxygen content is designated by z since the exact stoichiometric proportion of oxygen in CSVO can vary depending on whether the cathode material is prepared in an oxidizing atmosphere such as air or oxygen, or in an inert atmosphere such as argon, nitrogen and helium. For a more detailed description of this cathode active material reference is made to U.S. Patent Nos. 5,472,810 to Takeuchi et al. and 5,516,340 to Takeuchi et al., both of which are assigned to the assignee of the present invention and incorporated herein by reference.

According to the present invention, the metal vanadium oxide active material has an average particle size of less than 1µ and, more preferably, having an average diameter of from about 5 nanometers (nm) to about 100 nm. Still more preferably, the first active material has an average particle size of about 5 nm to about 50 nm. Preferably, the active particles have a very narrow distribution of particle diameters without a tail. In other words, there are effectively no particles with a diameter an order of magnitude greater than the average diameter such that the particle size distribution rapidly drops to zero.

The cathode design of the present invention further includes a second active material of a relatively high energy density and a relatively low rate capability in comparison to the first cathode active material. The second active material is preferably a carbonaceous compound prepared from carbon and fluorine, which includes graphitic and nongraphitic forms of carbon, such as coke, charcoal or activated carbon. Fluorinated carbon is represented by the formula (CFₓ)ₙ wherein x varies between about 0.1 to 1.9 and preferably between about 0.2 and 1.2, and (C₂F)ₙ wherein the n refers to the number of monomer units which can vary widely. The true density of CFₓ is 2.70 g/ml and its theoretical capacity is 2.42 Ah/ml.

In a broader sense, it is contemplated by the scope of the present invention that the first cathode active material is any material that has a relatively lower energy density but a relatively higher rate capability than the second active material. In addition to silver vanadium oxide and copper silver vanadium oxide, V₂O₅, MnO₂, LiCoO₂, LiNiO₂, LiMn₂O₄, TiS₂, Cu₂S, FeS, FeS₂, copper oxide, copper vanadium oxide, and mixtures thereof are useful as the first active material. And, in addition to fluorinated carbon, Ag₂O, Ag₂O₂, CuF, Ag₂CrO₄, MnO₂, and even SVO itself (although the first active and second active materials may not simultaneously be SVO unless at least the relatively high rate capability material is nanoparticulate; that is, has an average diameter less than about 1 µm), are useful as the second active material. The theoretical volumetric capacity (Ah/ml) of CFₓ is 2.42, Ag₂O₂ is 3.24, Ag₂O is 1.65 and AgV₂O_{5.5} is 1.37. Thus, CFₓ, Ag₂O₂, Ag₂O, all have higher theoretical volumetric capacities than that of SVO.

Before fabrication into an electrode structure for incorporation into an electrochemical cell according to the present invention, the first cathode active material is preferably mixed with a binder material such as a powdered fluoro-polymer, more preferably powdered polytetrafluoroethylene or powdered polyvinylidene fluoride present at about 1 to about 5 weight percent of the cathode mixture. Further, up to about 10 weight percent of a conductive diluent is preferably added to the first cathode mixture to improve conductivity. Suitable materials for this purpose include acetylene black, carbon black and/or graphite or a metallic powder such as powdered nickel, aluminum, titanium and stainless steel. The preferred first cathode active mixture thus includes a powdered fluoro-polymer binder present at about 3 weight percent, a conductive diluent present at about 3 weight percent and about 94 weight percent of the metal vanadium oxide active material.

The second cathode active mixture includes a powdered fluoro-polymer binder present at about 4 weight percent, a conductive diluent present at about 5 weight percent and about 91 weight percent of the cathode active material. A preferred second active mixture is, by weight, 91% CFₓ, 4% PTFE and 5% carbon black.

Cathode components for incorporation into an electrochemical cell according to the present invention may be prepared by rolling, spreading or pressing the first and second cathode active materials onto a suitable current collector selected from the group consisting of stainless steel, titanium, tantalum, platinum and gold. The preferred current collector material is titanium, and most preferably the titanium cathode current collector has a thin layer of graphite/carbon paint applied thereto. Still another preferred method for contacting the metal vanadium oxide nanoparticles to the current collector is described in U.S. Patent No. 5,716,422 to Muffoletto et al. This patent, which is assigned to the assignee of the present invention, describes various thermal-spraying processes and is incorporated herein by reference. Cathodes prepared as described above may be in the form of one or more plates operatively associated with at least one or more plates of anode material, or in the form of a strip wound with a corresponding strip of anode material in a structure similar to a "jellyroll".

According to the present invention, SVO cathode material, which provides a relatively high power or rate capability but a relatively low energy density or volumetric capability and CFₓ cathode material, which has a relatively high energy density but a relatively low rate capability, are individually contacted to current collector screens. This provides both materials in direct contact with the current collector. Therefore, one exemplary cathode plate for a primary cell has the following configuration:
SVO/current collector/CFₓ/current collector/SVO

An important aspect of the present invention is that the high rate cathode material (in this case the SVO material) maintains direct contact with the current collector. Another embodiment of the present invention has the high capacity/low rate material sandwiched between the high rate cathode material, in which the low rate/high capacity material is in direct contact with the high rate material. This cathode design has the following configuration:
SVO/current collector/SVO/CFₓ/SVO/current collector/SVO

Another important aspect of the present invention is that the high capacity material having the low rate capability is preferably positioned between two layers of high rate cathode material (either high or low capacities). In other words, the exemplary CFₓ material never directly faces the lithium anode. In addition, the low rate cathode material must be short circuited with the high rate material, either by direct contact as demonstrated above in the second embodiment, or by parallel connection through the current collectors as in the first illustrated embodiment above.

Since CFₓ material has significantly higher volumetric capacity than that of SVO material, i.e., approximately 1.77 times greater, in order to optimize the final cell capacity, the amount of CFₓ material should be maximized and the amount of SVO material used in each electrode should be minimized to the point that it is still practical in engineering and acceptable in electrochemical performance.

Further, end of service life indication is the same as that of a standard Li/SVO cell. And, it has been determined that the SVO electrode material and the CFₓ electrode material according to the present invention reach end of life at the same time. This is the case in spite of the varied usage in actual defibrillator applications. Since both electrode materials reach end of service life at the same time, no energy capacity is wasted.

A secondary cell according to the present invention takes advantage of active materials that are typically used as cathode active materials in primary cells, but which cannot normally be used in conventional secondary cells. The current art in rechargeable cells is to use the positive electrode as the source of alkali metal ions. This prohibits the use of metal-containing active materials that do not contain alkali metal ions. Examples of such metal-containing materials include V₂O₅, V₆O₁₃, silver vanadium oxide (SVO), copper silver vanadium oxide (CSVO), MnO₂, TiS₂, MoS₂, NbSe₃, CuO₂, Cu₂S, FeS, FeS₂, CFₓ, Ag₂O, Ag₂O₂, CuF, Ag₂CrO₄, copper oxide, copper vanadium oxide, and mixtures thereof.

However, the positive electrode of the present secondary cells is built in a double current collector configuration having a "sacrificial" piece of alkali metal, preferably lithium, sandwiched between the current collectors. A cathode active material capable of intercalation and de-intercalation the alkali metal contacts the opposite side of at least one, and preferably both, of the current collectors. The purpose of the sacrificial alkali metal is to react with the cathode active material upon the cell being activated with an electrolyte. The reaction results in a lithiated cathode active material.

Suitable current collectors are similar to those useful in the negative electrode and selected from copper, stainless steel, titanium, tantalum, platinum, gold, aluminum, nickel, cobalt nickel alloy, highly alloyed ferritic stainless steel containing molybdenum and chromium, and nickel-, chromium-, and molybdenum-containing alloys. Preferably the current collector is a perforated foil or screen, such as an expanded screen.

Preferred embodiments include the following positive electrode configurations:
vanadium oxide/current collector/lithium/current collector/vanadium oxide, or
vanadium oxide/current collector/vanadium oxide/lithium/vanadium oxide/current collector/vanadium oxide, or
vanadium oxide/current collector/lithium, with the vanadium oxide facing the negative electrode.

By the term "vanadium oxide" is meant V₂O₅, V₆O₁₃, silver vanadium oxide, and copper silver vanadium oxide in a nanoparticle form.

With this double current collector electrode design, the amount of lithium metal is adjusted to fully lithiate the cathode active material. Upon activating the cell with an ion-conductive electrolyte, the alkali metal migrates into the cathode active material resulting in complete consumption of the alkali metal. The absence of the alkali metal in the cell preserves the desirable safety and cycling properties of the intercalation negative and positive electrodes.

The anode or negative electrode for the secondary cell comprises an anode material capable of intercalating and de-intercalating lithium. Typically, the anode material of the negative electrode comprises any of the various forms of carbon (e.g., coke, graphite, acetylene black, carbon black, glassy carbon, etc.) that are capable of reversibly retaining the lithium species. Graphite is particularly preferred in conventional secondary cells. "Hairy carbon" is another particularly preferred conventional material due to its relatively high lithium-retention capacity. "Hairy carbon" is a material described in U.S. Patent No. 5,443,928 to Takeuchi et al., which is assigned to the assignee of the present invention and incorporated herein by reference.

The negative electrode for a secondary cell is fabricated by mixing about 90 to 97 weight percent of the carbonaceous anode material with about 3 to 10 weight percent of a binder material, which is preferably a fluoro-resin powder such as polytetrafluoroethylene (PTFE), polyvinylidene fluoride (PVDF), polyethylenetetrafluoroethylene (ETFE), polyamides, polyimides, and mixtures thereof. This negative electrode admixture is provided on a current collector selected from copper, stainless steel, titanium, tantalum, platinum, gold, aluminum, nickel, cobalt nickel alloy, highly alloyed ferritic stainless steel containing molybdenum and chromium, and nickel-, chromium-, and molybdenum-containing alloys. The current collector is a foil or screen and contact is by casting, pressing, or rolling the admixture thereto.

Another type of anode material useful with the present invention is a metal that reversibly alloys with alkali metals. Such metals include, but are not limited to, Sn, Si, Al, Pb, Zn, Ag, SnO, SnO₂, SiO, and SnO(B₂O₃)ₓ(P₂O₅)y. For a more detailed description of the use of these materials in the negative electrode of a secondary cell, reference is made to U.S. application Serial No. 10/008,977, filed November 8, 2001, which is assigned to the assignee of the present invention and incorporated herein by reference.

In order to prevent internal short circuit conditions, the cathode is separated from the anode by a suitable separator material. The separator is of electrically insulative material, and the separator material also is chemically unreactive with the anode and cathode active materials and both chemically unreactive with and insoluble in the electrolyte. In addition, the separator material has a degree of porosity sufficient to allow flow there through of the electrolyte during the electrochemical reaction of the cell. Illustrative separator materials include fabrics woven from fluoropolymeric fibers including polyvinylidine fluoride, polyethylenetetrafluoroethylene, and polyethylenechlorotrifluoroethylene used either alone or laminated with a fluoropolymeric microporous film, non-woven glass, polypropylene, polyethylene, glass fiber materials, ceramics, polytetrafluoroethylene membrane commercially available under the designation ZITEX (Chemplast Inc.), polypropylene/polyethylene membrane commercially available under the designation CELGARD (Celanese Plastic Company, Inc.), a membrane commercially available under the designation DEXIGLAS (C.H. Dexter, Div., Dexter Corp.), and a polyethylene membrane commercially available from Tonen Chemical Corp.

The primary electrochemical cell further includes a nonaqueous electrolyte that exhibits those physical properties necessary for ionic transport, namely, low viscosity, low surface tension and wettability. The electrolyte has an inorganic, ionically conductive salt dissolved in a mixture of aprotic organic solvents comprising a low viscosity solvent and a high permittivity solvent. In the case of an anode comprising lithium, preferred lithium salts that are useful as a vehicle for transport of alkali metal ions from the anode to the cathode include LiPF₆, LiBF₄, LiAsF₆, LiSbF₆, LiClO₄, LiO₂, LiAlCl₄, LiGaCl₄, LiC(SO₂CF₃)₃, LiN(SO₂CF₃)₂, LiSCN, LiO₃SCF₃, LiC₆F₅SO₃, LiO₂CCF₃, LiSO₆F, LiB(C₆H₅)₄ and LiCF₃SO₃, and mixtures thereof.

Low viscosity solvents useful with the present invention include esters, linear and cyclic ethers and dialkyl carbonates such as tetrahydrofuran (THF), methyl acetate (MA), diglyme, trigylme, tetragylme, dimethyl carbonate (DMC), 1,2-dimethoxyethane (DME), 1,2-diethoxyethane (DEE), 1-ethoxy,2-methoxyethane (EME), ethyl methyl carbonate, methyl propyl carbonate, ethyl propyl carbonate, diethyl carbonate, dipropyl carbonate, and mixtures thereof, and high permittivity solvents include cyclic carbonates, cyclic esters and cyclic amides such as propylene carbonate (PC), ethylene carbonate (EC), butylene carbonate, acetonitrile, dimethyl sulfoxide, dimethyl formamide, dimethyl acetamide, γ-valerolactone, γ-butyrolactone (GBL), N-methyl-pyrrolidone (NMP), and mixtures thereof. In the present invention, the preferred anode is lithium metal and the preferred electrolyte is 0.8M to 1.5M LiAsF₆ or LiPF₆ dissolved in a 50:50 mixture, by volume, of propylene carbonate and 1,2-dimethoxyethane.

A preferred electrolyte for a secondary cell comprises a solvent mixture of EC:DMC:EMC:DEC. Most preferred volume percent ranges for the various carbonate solvents include EC in the range of about 20% to about 50%; DMC in the range of about 12% to about 75%; EMC in the range of about 5% to about 45%; and DEC in the range of about 3% to about 45%. In a preferred form, the electrolyte is at equilibrium with respect to the molar ratio of DMC:EMC:DEC. This electrolyte is described in detail in U.S. patent application Serial No. 10/232,166, filed August 30, 2002, which is assigned to the assignee of the present invention and incorporated herein by reference.

The corrosion resistant glass used in the glass-to-metal seals has up to about 50% by weight silicon such as CABAL 12, TA 23, FUSITE 425 or FUSITE 435. The positive terminal leads preferably comprise molybdenum, although titanium, aluminum, nickel alloy, or stainless steel can also be used. The cell casing is an open container hermetically sealed with a lid typically of a material similar to that of the casing.

It is contemplated that both the present invention primary and secondary cells are capable of serving as the power source for a wide range of implantable medical devices. These include a cardiac pacemaker, a cardiac defibrillator, a neuro-stimulator, a drug delivery system, a bone-healing implant, and a hearing implant.

The following examples describe the manner and process of the present invention, and they set forth the best mode contemplated by the inventors of carrying out the invention, but they are not to be construed as limiting.

### EXAMPLE I

SVO was synthesized using LiOH, AgNO₃ and V₂O₅, in a ratio of 0.05:0.95:2.0.. A 23.03-gram sample of V₂O₅ was mixed with 10.23 grams of AgNO₃ and 0.0075 grams of LiOH to give 33.33 grams of total solids. The mixture was added to 100 ml of distilled water to form a slurry that was 25% solids and/or dissolved solids per solution weight. The slurry was heated to about 90°C for about 3 hours with stirring. After about 30 minutes to 1 hour, the solids appeared to have absorbed all of the solvent and expanded to the full volume of the mixture. The mixture was the consistency of a thick orange/red paste. The sample was then cooled prior to dehydration and sintering at about 375°C for about 24 hours under ambient atmosphere.

The dehydrated SVO material was ground lightly using a mortar and pestle giving an orange/brown powder. The resulting solid material was imaged using an SEM. Average particle size is less than 1 micron.

### EXAMPLE II

Silver vanadium oxide nanoparticles can be plasma spray deposited in air using a Metco 3MB machine on a setting of 40 liters/minute of argon as the principle gas and 2.5 liters/minute (nominal) of hydrogen as the secondary gas. This mixture is directed through a 50-volt/400-amp direct current arc. A suitable spray distance is 3 inches using 4 liters/minute of carrier gas for the electrode active material having a nominal feed rate of 40 grams/minute. A suitable substrate is 0.0045 inches thick titanium foil, cleaned and mirogrit blasted (particle size about 80 microns). The spray deposited SVO nanoparticles are expected to have an average size of about 50 nm to about 500 nm.

It is appreciated that various modifications to the inventive concepts described herein may be apparent to those of ordinary skill in the art without departing from the spirit and scope of the present invention as defined by the appended claims.

## Claims

1. An electrochemical cell, which comprises:
a) an anode of an alkali metal;
b) a cathode of a first cathode active material having a relatively high energy density but a relatively low rate capability short circuited with a second cathode active material having a relatively low energy density but a relatively high rate capability; and
c) a nonaqueous electrolyte activating the anode and the cathode, wherein at least the second cathode active material is of particles having an average diameter less than about 1 µm.

2. The electrochemical cell of Claim 1 wherein, in b), a cathode of a first cathode active material having a relatively high energy density but a relatively low rate capability is sandwiched between first and second current collectors with a second cathode active material having a relatively low energy density but a relatively high rate capability contacting the first and second current collectors opposite the first cathode active material, wherein at least the second cathode active material is of particles having an average diameter less than about 1 µm.

3. The electrochemical cell of Claim 1 or Claim 2 wherein at least the second cathode active material is of particles having an average diameter of 5 nanometers to 50 nanometers.

4. The electrochemical cell of any one of the preceding claims wherein the first cathode active material is selected from the group consisting of CFₓ, Ag₂O, Ag₂O₂, CuF₂, Ag₂CrO₄, MnO₂, SVO, and mixtures thereof.

5. The electrochemical cell of any one of the preceding claims wherein the second cathode active material is selected from the group consisting of SVO, CSVO, V₂O₅, MnO₂, LiCoO₂, LiNiO₂, LiMnO₂, CuO₂, TiS, Cu₂S, FeS, FeS₂, copper oxide, copper vanadium oxide, and mixtures thereof.

6. The electrochemical cell of any one of the preceding claims wherein the cathode has the configuration: SVO/current collector/CFₓ/current collector/SVO.

7. The electrochemical cell of any one of the preceding Claims 1 to 5 wherein the cathode has the configuration: SVO/current collector/SVO/CFₓ/SVO/current collector/SVO.

8. The electrochemical cell of any one of the preceding Claims 1 to 5 wherein the cathode has the configuration: SVO/current collector/CFₓ, with the SVO facing the anode.

9. The electrochemical cell of any one of the preceding Claims 2 to 8 wherein the first and second current collectors are selected from the group consisting of stainless steel, titanium, tantalum, platinum and gold.

10. The electrochemical cell of Claim 9 wherein the first and second current collectors are titanium having a graphite/carbon material coated thereon.

11. The electrochemical cell of any one of the preceding Claims 2 to 10 wherein the anode is lithium, the first cathode active material is CFₓ, the second cathode active material is SVO and the first and second current collectors are titanium.

12. An electrochemical cell which comprises:
a) a negative electrode of an anode material;
b) a positive electrode of a cathode active material short circuited with an anode active material; and
c) a nonaqueous electrolyte activating the negative electrode and the positive electrode.

13. The electrochemical cell of Claim 12 wherein the cathode active material is of particles having an average diameter less than about 1µm.

14. The electrochemical cell of Claim 12 or Claim 13 wherein at least the second cathode active material is of particles having an average diameter of 5 nanometers to 50 nanometers.

15. The electrochemical cell of any one of the preceding Claims 12 to 14 wherein the cathode active material is selected from the group consisting of V₂O₅, V₆O₁₃, SVO, CSVO, MnO₂, TiS₂, MoS₂, NbSe₃, CuO₂, Cu₂S, FeS, FeS₂, CFₓ, Ag₂O, Ag₂O₂, CuF₂, Ag₂CrO₄, copperoxide, coppervanadium oxide, polypyrroles, polythiophenes, polysulfides, polyanilines, polyacetylenes, and mixtures thereof.

16. The electrochemical cell of any one of the preceding Claims 12 to 15 wherein the anode material is selected from the group consisting of coke, graphite, acetylene black, carbon black, glassy carbon, hairy carbon, hard carbon, Sn, Si, Al, Pb, Zn, Ag, SnO, SnO₂, SiO, SnO(B₂O₃)ₓ(P₂O₅)_{y}, and mixtures thereof.

17. The electrochemical cell of any one of the preceding Claims 12 to 16 wherein the positive electrode has the configuration: first cathode active material/current collector/alkali metal/current collector/second cathode active material, wherein the first and second cathode active materials are capable of intercalating and de-intercalating the alkali metal and are the same or different.

18. The electrochemical cell of any one of the preceding Claims 12 to 16 wherein the positive electrode has the configuration: first cathode active material/current collector/second cathode active material/alkali metal/third cathode active material/current collector/fourth cathode active material, wherein the first, second, third and fourth cathode active materials are capable of intercalating and de-intercalating the alkali metal and are either the same or different.

19. The electrochemical cell of any one of the preceding Claims 12 to 16 wherein the positive electrode has the configuration: cathode active material/current collector/alkali metal, wherein the cathode active material is capable of intercalating and de-intercalating the alkali metal.

20. The electrochemical cell of Claim 19 wherein the cathode active material faces the negative electrode.

21. The electrochemical cell of any one of the preceding Claims 12 to 17 wherein the cathode active material is a vanadium oxide and the positive electrode has the configuration: vanadium oxide/current collector/lithium/current collector/vanadium oxide.

22. The electrochemical cell of any one of the preceding Claims 19 or 20 wherein the cathode active material is a vanadium oxide and the alkali metal is lithium.

23. The electrochemical cell of any one of the preceding Claims 12 to 18 wherein the cathode active material is a vanadium oxide and the positive electrode has the configuration: vanadium oxide/current collector/vanadium oxide/lithium/vanadium oxide/current collector/vanadium oxide.

24. The electrochemical cell of any one of the preceding claims wherein the electrolyte is 0.8M to 1.5M LiAsF₆ or LiPF₆ dissolved in a 50:50 mixture, by volume, of propylene carbonate as the first solvent and 1,2-dimethoxyethane as the second solvent.

25. An implantable medical device in combination with an electrochemical cell of any one of the preceding claims powering the medical device.

26. The combination of Claim 25 wherein the anode is lithium, the first cathode active material is CFₓ, the second cathode active material is SVO.

27. The combination of Claim 26 including providing the anode of lithium and the cathode having the configuration: SVO/current collector/CFₓ, with the SVO facing the lithium anode.

28. The combination of any one of Claims 25, 26 or 27 wherein the implantable medical device is selected from the group consisting of a cardiac pacemaker, a cardiac defibrillator, a neuro-stimulator, a drug delivery system, a bone-healing implant, and a hearing implant.

29. A method for providing an electrochemical cell, comprising the steps of:
a) providing a negative electrode of an anode material;
b) providing a positive electrode of an alkali metal short circuited with a cathode active material; and
c) activating the negative electrode and the positive electrode with a nonaqueous electrolyte.

30. The method of Claim 29 wherein the electrode materials and/or the electrode configurations are defined in any one of Claims 1 to 22.

31. The method of Claim 29 or 30 including providing at least the first cathode active material by a process selected from the group consisting of sol-gel synthesis, hydrothermal synthesis, combustion chemical vapor deposition, laser pyrolysis, a decomposition reaction, and a combination reaction.

32. The method of Claim 29, 30 or 31 including providing the cathode active material as a vanadium oxide selected from the group consisting of V₂O₅, V₆O₁₃, silver vanadium oxide, copper silver vanadium oxide, and mixtures thereof.

33. Use of a high rate capability cathode active material, in nanoparticulate form, to improve discharge capability of an electrochemical cell including an electrode comprising such material.
